(19) Europäisches Patentamt
European Patent Office
Office européen des brevets

(11) **EP 4 592 385 A1**

(12) **EUROPEAN PATENT APPLICATION**
published in accordance with Art. 153(4) EPC

(43) Date of publication:
30.07.2025 Bulletin 2025/31

(21) Application number: 23917007.9

(22) Date of filing: 17.07.2023

(51) International Patent Classification (IPC):
*C12N 9/06* (2006.01)     *C12N 15/70* (2006.01)
*C12N 1/21* (2006.01)     *C12P 17/10* (2006.01)
*C12R 1/19* (2006.01)

(52) Cooperative Patent Classification (CPC):
C12N 9/0004; C12N 15/70; C12N 15/74;
C12P 17/10; C12R 2001/19; Y02P 20/55

(86) International application number:
PCT/CN2023/107618

(87) International publication number:
WO 2024/152535 (25.07.2024 Gazette 2024/30)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 16.01.2023 CN 202310056157

(71) Applicant: Zhejiang Ano Bio-Pharmaceutical Co.,
Ltd.
Hangzhou, Zhejiang 310018 (CN)

(72) Inventors:
• WANG, Yajun
  Hangzhou, Zhejiang 310018 (CN)
• HU, Yongwei
  Hangzhou, Zhejiang 310018 (CN)

(74) Representative: Ipside
7-9 Allée Haussmann
33300 Bordeaux Cedex (FR)

Remarks:
The complete document including Reference
Table(s) and the Sequence Listing(s) can be
downloaded from the EPO website

(54) **IMINE REDUCTASE MUTANT, AND USE THEREOF AND USE METHOD THEREFOR**

(57) The present invention belongs to the technical field of biological catalysis and organic synthesis, and in particular, relates to an imine reductase mutant, application thereof and an application method therefor. According to the present invention, the imine reductase mutant has an amino acid sequence derived by a mutation occurring to an amino acid sequence as set forth in SEQ ID NO. 1, and an optional mutation site includes one of the following sites: position 44 with G mutating into V, or position 89 with L mutating into V, or positions 44 and 89 with G and L simultaneously mutating into V, respectively. The imine reductase mutant of the present invention binds to glucose dehydrogenase to prepare an intermediate (S)-2-methyl-5-(pyrrol-2-yl)pyridine through asymmetric hydrogenation reduction by taking β-nicotinamide adenine dinucleotide disodium salt as a coenzyme and 5-(3,4-dihydro-2H-pyrrol-5-yl)-2-methylpyridine as a substrate, with a chemical conversion rate up to above 99.5% and e.e.% up to 99.6%, which provides a production basis for the industrialization of (S)-2-methyl-5-(methylpyrrol-2-yl)pyridine and shows good industrial application value.

EP 4 592 385 A1

## Description

## Background of the Invention

1. Technical Field

The present invention belongs to the technical field of biological catalysis and organic synthesis, and in particular, relates to an imine reductase mutant, application thereof and an application method therefor.

2. Description of Related Art

Neurodegenerative diseases are one of the major threats to human health. With the continuous in-depth study on such diseases, some scholars believe that nicotinic acetylcholine receptors (nAChR) in neurons are important targets for drug therapies (Neuropharmacology, 1995, 34, 563). Many studies have demonstrated that natural alkaloid nicotine had an effect of alleviating and treating Parkinson's disease, Alzheimer's disease and Tourette to some extent, but showed toxic and side effects on the cardiovascular system and digestive system, which greatly limits its clinical application (Chem Eng. News 2000, 78, 23-26). Existing literature (J. Med. Chem. 1997, 40, 4169; J.Pharm.Exp.Ther. 2000, 292, 461) summarized the structure-activity relationship of nAChR ligands through systematic studies. As early as the end of the twentieth century, some new nicotine analogues had been discovered and applied to the clinical treatment of Parkinson's disease (j. org. chem. 1999, 64, 5299). Dukat et al. (Eur. J. Med. Chem. 1999, 34, 31) summarized and reported that the affinity of substituted nicotine to nAChR receptors was related to the lipophilicity of a 6-position substituent. Related studies further showed that the affinity decreased as the size of the substituent increased. That is, the affinity of nicotine analogues is determined by the lipophilicity of the 6-position substituent, and also related to the size of the substituent. Here, the 6-position substituent of 6-methyl nicotine has the smallest size and strongest lipophilicity, and has strong affinity with nAChR receptors, showing a good medicinal prospect. The prior art is almost concentrated in racemate synthesis, and there is no report on the synthesis of single configuration.

It is the striving direction of enzymic industrial production of (S)-2-methyl-5-(pyrrol-2-yl)pyridine to find out imine reductases from different biological sources and modify enzyme molecules with the help of protein engineering methods to obtain more efficient imine reductases.

## Summary of the Invention

An object of the present invention is to provide an imine reductase mutant, which can convert 5-(3,4-dihydro-2H-pyrrol-5-yl-2-methylpyridine into a single chiral intermediate (S)-2-methyl-5-(pyrrol-2-yl)pyridine through asymmetric hydrogenation reduction, with a chemical conversion rate up to above 99.5% and e.e.% up to 99.6%, providing a production basis for the industrialization of (S)-2-methyl-5-(methylpyrrol-2-yl)pyridine and showing good industrial application value.

According to the present invention, on the basis of a wild type imine reductase with an amino acid sequence as set forth in SEQ NO. 1 and a nucleotide sequence as set forth in SEQ NO. 5, the imine reductase is mutated with a molecular biological method of random mutation to change the amino acid sequence of an enzyme and thus the structure and function of the enzyme, and then, a directed screening method is performed to obtain three types of imine reductase mutants, each with an amino acid sequence derived by mutation of G on position 44 into V, mutation of L on position 89 into L, or simultaneous mutation of G and L on positions 44 and 89 into V, respectively, on the basis of the amino acid sequence as set forth in SEQ NO. 1.

Specifically, the amino acid sequence of the imine reductase mutant of the present invention is as set forth in SEQ NO. 2, SEQ NO. 3, or SEQ NO. 4.

Specifically, the nucleotide sequence of SEQ NO. 2 of the imine reductase mutant of the present invention is as set forth in SEQ ID NO. 6.

Specifically, the nucleotide sequence of SEQ NO. 3 of the imine reductase mutant of the present invention is as set forth in SEQ ID NO. 7.

Specifically, the nucleotide sequence of SEQ NO. 4 of the imine reductase mutant of the present invention is as set forth in SEQ ID NO. 8.

The present invention further provides a recombinant plasmid comprising a coding gene of the imine reductase mutant with the amino acid sequence as set forth in SEQ NO. 2, SEQ NO. 3, or SEQ NO. 4.

The present invention further provides a host cell comprising the recombinant plasmid. The host cell is selected from a prokaryotic cell or an eukaryotic cell, and the prokaryotic cell is an *Escherichia coli* BL21 (DE3) cell.

The present invention further provides application of the imine reductase mutant described above in synthesis of an intermediate (S)-2-methyl-5-(pyrrol-2-yl)pyridine.

An application method includes: binding an imine reductase mutant to glucose dehydrogenase to prepare an intermediate (S)-2-methyl-5-(pyrrol-2-yl)pyridine through asymmetric hydrogenation reduction by taking β-nicotinamide adenine dinucleotide disodium salt as a coenzyme and 5-(3,4-dihydro-2H-pyrrol-5-yl)-2-methylpyridine as a substrate.

Preferably, during the preparation of the intermediate (S)-2-methyl-5-(pyrrol-2-yl)pyridine through asymmetric hydrogenation reduction, a pH value is 6.8-8.0.

Further preferably, the pH value is 7.2-7.6, and the pH value during reaction is regulated by taking a sodium hydroxide aqueous solution with a mass concentration of 8%, as a pH regulator. During reaction where the pH value is lower than 6.8 or higher than 8.0, the reaction conversion rate and the reaction speed are significantly decreased.

Preferably, during the preparation of the intermediate (S)-2-methyl-5-(pyrrol-2-yl)pyridine from the imine reductase mutant, a reaction temperature is controlled to 15-35°C during reaction.

Further preferably, the reaction temperature is in a range of 25-30°C, and when the temperature is lower than 15°C or higher than 35°C, the reaction conversion rate and the reaction speed are significantly decreased.

Compared with the prior art, the present invention has the following advantageous effects:

1) when used in the catalysis of 5-(3,4-dihydro-2H-pyrrol-5-yl)-2-methylpyridine for asymmetric hydrogenation reduction, the imine reductase mutant of the present invention has the enzymatic activity above 10 times higher than the wild type imine reductase, and achieves very high chemical conversion rate and stereoselectivity; and

2) when used in the preparation of the intermediate catalysis of (S)-2-methyl-5-(pyrrol-2-yl)pyridine, the imine reductase mutant of the present invention has the chemical conversion rate up to above 99.5% and e.e.% up to 99.6%, providing a production basis for the industrialization of (S)-2-methyl-5-(methylpyrrol-2-yl)pyridine and showing good industrial application value.

## Detailed Description of Embodiments

The present invention is explained in detail below in combination with embodiments.

Example 1: Acquisition of Recombinant Plasmid of Wild Type Parent Imine Reductase from *Actinomadura sp.* CNU-125 (*Actinomadura madurae*) Strain

Based on the NCBI Genbank nucleic acid database and through codon optimization, full-length genes were artificially synthesized into pET21a(+) expression plasmids by a commissioned service provider, and these pET21 a(+) expression plasmids were transformed into Escherichia coli BL21(DE3) competent cells, coated on LB agar plates containing 100 mg/L ampicillin, and cultured at 37°C for 24 hours. Several single colonies were selected and cultured at 37°C for 24 hours in the LB medium containing 100 mg/L ampicillin, and then the recombinant plasmids were extracted using the plasmid mini-extraction kit for PCR and sequencing verification, such that the recombinant plasmids of imine reductases were obtained.

Example 2: Random Mutation of Genes of Wild Type Parent Imine Reductase

Based on the description in Example 1, the recombinant plasmid containing the coding gene of the parent imine reductase of the *Actinomadura sp.* strains was used as a template, and primers at both ends (Table 1) were designed and synthesized with Primer 5.0 based on the coding gene of the parent imine reductase of *Actinomadura sp.* strains. Linear gene fragments with a large number of base mutations were obtained by using the error-prone PCR technique (see Table 2 for material concentration and Table 3 for reaction conditions). Then, these RCP products and the pET21a(+) expression plasmids were digested, gel-extracted, ligated, transformation into *Escherichia coli* BL21 (DE3) competent cells, coated on LB agar plates with 100 mg/L ampicillin, and cultured at 37°C for 24 hours.

Table 1. Random mutation primer sequences

| Primer name | Primer sequence |
|---|---|
| Forward Primer | 5' ACATACATGTAATGAAGCCAGACGGTGTGA 3' |
| Reverse Primer | 5' AGGTCCTAGCGTTAAGCCGCACGCAGTTCC 3' |

Table 2. 50 μL of error-prone PCR material system

| Material name | Quantity (μL) |
|---|---|
| Parent recombinant plasmid (2 ng/μL) | 0.5 |

(continued)

| Material name | Quantity (μL) |
|---|---|
| Forward Primer (10 nM) | 1.5 |
| Reverse Primer (10 nM) | 1.5 |
| MnCl dedicated for error-prone PCR | 2.5 |
| MgCl dedicated for error-prone PCR | 2.5 |
| dNTP.10X dedicated for error-prone PCR | 5 |
| Mix 2.0 10X for error-prone PCR | 5 |
| Ultrapure water | 28 |
| Taq DNA polymerase (5 U/μL) | 4 |

Table 3. Reaction conditions for error-prone PCR

| Temperature | Time | Number of cycles |
|---|---|---|
| 95°C | 5 min | |
| 95°C | 12 s | 30 |
| 63°C | 12 s | 30 |
| 72°C | 60 s | 30 |
| 72°C | 10 min | |

Example 3: Cloning and Expression of Imine Reductase Mutant

Compatible restriction endonuclease cleavage points were designed at the 5'- and 3'-ends of the imine reductase gene to facilitate the cloning, expression and identification of the imine reductase mutants. The target genes and pET21a(+) could be simultaneously digested by Nde I and Xho I restriction endonucleases and then subjected to DNA gel extraction. Larger fragments of the recovered target genes and plasmids were ligated with T4 DNA ligases to transform serial products into *Escherichia coli* BL21 (DE3) competent cells. Then, the transformed competent cells were coated on LB agar plates containing 100 mg/L ampicillin and cultured at 37°C for 24 hours. Single colonies grown on the above plates were selected and cultured over shaking at 37°C for 24 hours in LB liquid medium with 100 mg/L ampicillin. Bacterial cells were collected for plasmid extraction, PCR identification and double enzyme digestion identification. Correct recombinant plasmids were renamed, and then subjected to the subsequent induced expression. The above bacterial solution was transferred to 500 mL of LB liquid medium containing 100 mg/L ampicillin and cultured over shaking at 37°C. When OD600 = 0.6-0.8, IPTG was added to induce the expression at 20-25°C for 18-24 hours. The bacterial solution was removed, and the resultant was then centrifuged in a high-speed centrifuge for 20-30 minutes to collect bacterial cells, which was cryopreserved at -20°C for later use.

Example 4: Primary Screening of Imine Reductase Mutants

Based on the description of Examples 2 and 3, monoclonal strains from the above LB agar medium were seeded to a 96-deep well plate; 1 mL of 100mg/L ampicillin solution was added to each well in advance; the plate was cultured over shaking at 37°C for 4 hours; and an inducer IPTG was added for induction culture at 20-25°C for 12-16 hours. The pate was centrifuged for 20-30 minutes in a high-speed centrifuge to collect bacterial cells. Then, the bacterial cells were added to a phosphate buffer saline (with the concentration of 100 mM) with the pH of 7, and crushed by a high-flux ultrasonic cell crusher, with a crushing time of 3 seconds at an interval of 3 seconds, at the temperature of 0-4°C. Then, high-speed centrifugation was carried out to obtain a supernatant, i.e., the crude enzyme solution of imine reductase mutants. Primary screening for activity was carried out using the microplate reader. 20 uL of 2 mmol/L reduced nicotinamide adenine dinucleotide (NADH), 100 uL of phosphate buffer solution (pH 7) with the concentration of 100 mM, 20 uL of enzyme solution, and 20 uL of 20mmol/L 5-(3,4-dihydro-2H-pyrrol-5-yl)-2-methylpyridine solution were added to a 96-well plate, and then detected for the change of A340 every 15 seconds within 5 minutes.

Note: Enzyme activity calculation formula:

$$\text{Enzyme activity (U/mL)} = (\Delta A \times V1 \times 1000)/(6220 \times t \times V2)$$

$\Delta A$: absorbance change value within t;
V1: total volume of reaction system, in mL;
V2: the volume of enzyme solution added, in mL;
6220: molar extinction coefficient, in L/mol/cm; and
t: detection time.

Example 5: Secondary Screening of Imine Reductase Mutants

Preparation of enzyme solution of imine reductase mutants: the mutants with high enzyme activity in Example 4 were seeded at 0.1% into 500 mL of LB medium containing 100 mg/L ampicillin solution and cultured over shaking at 37°C for 12-18 hours; an inducer IPTG was added for induction culture at 20-25°C for 16-24 hours; bacterial cells were collected after high-speed centrifugation, and redispersed in 100 mM phosphate buffer solution (pH 7); and the bacterial cells were homogenized at 0-5°C using a cell homogenizer under 600-800 bar to obtain the crude enzyme solution of imine reductase mutants.

Secondary screening of crude enzyme solution and catalytic reduction of 5-(3,4-dihydro-2H-pyrrol-5-yl)-2-methylpyridine: 0.1 g of 5-(3,4-dihydro-2H-pyrrol-5-yl)-2-methylpyridine was added to a 10 mL three-neck flask; 2 mL of purified water was added; 2 mg of β-nicotinamide adenine dinucleotide phosphate disodium salt was added; 0.5 mL of the crude enzyme solution of imine reductase mutants and 0.5 mL of crude solution of glucose dehydrogenase were added to react at 27°C for 24-48 hours, with the pH controlled to 7.0-7.5 during reaction; and the reaction conversion rate and ee% were analyzed, with screening results shown in Table 4.

Table 4. Comparison of activities of intermediates (S)-2-methyl-5-(pyrrol-2-yl)pyridine prepared with parent imine reductase and imine reductase mutants

| SEQ ID NO | | Mutation site | Enzyme quantity wt | Conversion rate % | ee % |
|---|---|---|---|---|---|
| Amino acid sequence | Nucleotide sequence | | | | |
| 1 | 5 | Parent | 4 | 30% | 97.9 |
| 2 | 6 | G43V | 0.6 | 98.8% | 99.1% |
| 3 | 7 | L89V | 0.6 | 99.1% | 99.3% |
| 4 | 8 | G43V-L89V | 0.6 | 99.5% | 99.6% |

Note: The enzyme quantity refers to the wet weight of the recombinant bacterial cells of imine reductase required to transform 1 g of substrate.

Example 6: Determination of Catalytic Conversion Rate of Imine Reductase

The reaction mixture of the reaction system described above was diluted 10-fold using 70% acetonitrile aqueous solution, the protein was removed by sedimentation, and membrane filtration was carried out, and the resultant was diluted 10-fold with purified water for injection.

Chromatographic condition: Agilent 1200 high performance liquid chromatograph;
Chromatographic column: XBridge C18, 4.6 x 150mm 3.5μM;
Mobile phase A: It was prepared by adding accurately weighed 1.5 g of glacial acetic acid to a 25 mL volumetric flask, diluting with purified water to a constant volume, adding the mixture to 900 mL of purified water, then regulating pH to 10.0 with 25% stronger ammonia water, and adding purified water to the mixture to a constant volume of 1000 mL; and
Mobile phase B: acetonitrile.

Chromatographic conditions:

| Detection wavelength: | 254 nm | | |
|---|---|---|---|
| Flow rate: | 1.0 mL/min | | |
| Injection volume: | 20 µL | | |
| Column temperature: | 30°C | | |
| Collection time: | 40 min | | |
| Gradient elution table | Time/min | Mobile phase A (%) | Mobile phase B (%) |
| | 0 | 100 | 0 |
| | 3 | 100 | 0 |
| | 3.01 | 95 | 5 |
| | 28 | 74 | 26 |
| | 32 | 60 | 40 |
| | 32.1 | 100 | 0 |
| | 43 | 100 | 0 |
| Chromatographic column washing procedures | Time/min | Flow rate (mL/min) | Water (%) | Acetonitrile (%) |
| | 0 | 0.5 | 95 | 5 |
| | 90 | 0.5 | 95 | 5 |
| | 100 | 0.5 | 5 | 95 |
| | 160 | 0.5 | 5 | 95 |

Conversion rate calculation formula: Conversion rate (%) = $A_P/(A_P + A_S)$ * 100

$A_P$: the peak area of product (S)-2-methyl-5-(pyrrol-2-yl)pyridine
$A_S$: the peak area of starting material 5-(3,4-dihydro-2H-pyrrol-5-yl)-2-methylpyridine

Example 7: Determination of Optical Purity of Imine Reductase-Catalyzed Substrate

The reaction mixture from Example 5 was concentrated and dried after posttreatment and extraction, and samples were taken and diluted with pure ethanol to formulate 2 mg/mL solution for injection.

Instrumentation: Agilent 1200 high performance liquid chromatograph
Chromatographic column: CHIRALPAK IC 250*4.6mm 5µm
Mobile phase A: It was prepared by accurately pipetting 2.0 mL of diethylamine to 980 mL of n-hexane and 20 mL of isopropanol, mixing them evenly and performing degassing.
Mobile phase B: n-hexane

Chromatographic conditions:

| Detection wavelength: | 260 nm | | |
|---|---|---|---|
| Flow rate: | 0.8 mL/min | | |
| Injection volume: | 15 μL | | |
| Column temperature: | 25°C | | |
| Collection time: | 60 min | | |
| Gradient elution table | Time/min | Mobile phase A (%) | Mobile phase B (%) |
| | 0 | 100 | 0 |
| | 37 | 100 | 0 |
| | 40 | 0 | 100 |
| | 40.1 | 100 | 0 |
| | 60 | 100 | 0 |

Note: Formula for calculating ee% of S-configuration product: ee (%) = $(A_S - A_R)/((A_S + A_R) * 100$
$A_S$: the peak area of product (S)-2-methyl-5-(pyrrol-2-yl)pyridine
$A_R$: the peak area of enantiomer (R)-2-methyl-5-(pyrrol-2-yl)pyridine

Example 8: Preparation of Enzyme Solution of Imine Reductase Mutants

The imine reductase mutant strains subjected to secondary screening in Example 5 were seeded at 0.01% in 10 500 mL flasks, in which 100 mg/L ampicillin LB medium was added in advance; the flasks were cultured over shaking at 37°C for 12-16 hours; a certain amount of inducer propyl-β-D-thiogalactoside (IPTG, with the final concentration of 0.1 mM) was added for induction culture at 20-25°C for 16-24 hours; and high-speed centrifugation was carried out to collect bacterial cells. 50 g of the bacterial cells obtained were resuspended in 100 g of purified water, and homogenized and crushed to obtain the crude enzyme solution of imine reductase mutants.

Example 9: Application of Imine Reductase Mutant as Set Forth in SEQ ID NO. 4 in Preparation of (S)-2-methyl-5-(pyrrol-2-yl)pyridine Through Substrate Reduction

50 g of 5-(3,4-dihydro-2H-pyrrol-5-yl)-2-methylpyridine was added to a 500 mL reaction flask, 150 mL purified water as a solvent and 123 g of dextrose monohydrate were added, and 90 mL of the enzyme solution of imine reductase mutants was added (the weight ratio of bacterial cells was 0.6). 90 mL of glucose dehydrogenase solution (the weight ratio of bacterial cells was 0.6) and 0.66 g of β-nicotinamide adenine dinucleotide phosphate disodium salt were added; the temperature was regulated to 27°C; and the pH change during reaction was monitored in real time and controlled to 2-3 with 8% sodium hydroxide aqueous solution. The conversion rate was 88.3% after 24-hour reaction, and 99.5% after 48-hour reaction. After the reaction was completed, the pH was regulated to 2-3 with an acid; then, 5 g of activated carbon and 5 g of diatomite were added; the reaction mixture was filtered; the pH of the filtrate was regulated to 13; then, methyl-tert-butyl extraction was carried; and organic phases were combined and concentrated to obtain a product, namely, 48.2 g of intermediate, with ee% of 99.6%.

The amino acid and nucleotide sequences of the above glucose dehydrogenases are as set forth in SEQ ID NO. 9 and SEQ ID NO. 10.

The amino acid and nucleotide sequences involved in the present invention are as follows.

SEQ ID NO. 1 (amino acid sequence of imine reductase):

MKTPVTVLGLGAMGRALVTALLDAGRPVTVWNRTPGKADELAGAVEAASVHDAVTAGGI
VVVCLYDHASVHETLDPVAAGLRGRAVVNLTTTTPAEARELAEWADGHGIEYLDGAIMAT
PPMIGAPGAQILYSGSRAVFDGHREALDLWATSVYDGADAGMASLFDLAILSGMYPLFAG
FLHGAAMVRAEGVPAAEFAERAAPFLAAMTGSFAGIARTVDGGGYDVPGQSLDWTAGA
LEAIGRASREQGVDPVPVDMVGALVRRQIDEGYGSEDMSRLIESMTGDAV

SEQ SEQ ID. 2 (amino acid sequence of imine reductase mutant G43V):

MKTPVTVLGLGAMGRALVTALLDAGRPVTVWNRTPGKADELAVAVEAASVHDAVTAGGI
VVVCLYDHASVHETLDPVAAGLRGRAVVNLTTTTPAEARELAEWADGHGIEYLDGAIMAT
PPMIGAPGAQILYSGSRAVFDGHREALDLWATSVYDGADAGMASLFDLAILSGMYPLFAG
FLHGAAMVRAEGVPAAEFAERAAPFLAAMTGSFAGIARTVDGGGYDVPGQSLDWTAGA
LEAIGRASREQGVDPVPVDMVGALVRRQIDEGYGSEDMSRLIESMTGDAV

SEQ ID NO. 3 (amino acid sequence of imine reductase mutant L89V):

MKTPVTVLGLGAMGRALVTALLDAGRPVTVWNRTPGKADELAGAVEAASVHDAVTAGGI
VVVCLYDHASVHETLDPVAAGLRGRAVVNVTTTTPAEARELAEWADGHGIEYLDGAIMAT
PPMIGAPGAQILYSGSRAVFDGHREALDLWATSVYDGADAGMASLFDLAILSGMYPLFAG
FLHGAAMVRAEGVPAAEFAERAAPFLAAMTGSFAGIARTVDGGGYDVPGQSLDWTAGA
LEAIGRASREQGVDPVPVDMVGALVRRQIDEGYGSEDMSRLIESMTGDAV

SEQ ID NO. 4 (amino acid sequence of imine reductase mutant G43V-L89V):

MKTPVTVLGLGAMGRALVTALLDAGRPVTVWNRTPGKADELAVAVEAASVHDAVTAGGI
VVVCLYDHASVHETLDPVAAGLRGRAVVNVTTTTPAEARELAEWADGHGIEYLDGAIMAT

PPMIGAPGAQILYSGSRAVFDGHREALDLWATSVYDGADAGMASLFDLAILSGMYPLFAG
FLHGAAMVRAEGVPAAEFAERAAPFLAAMTGSFAGIARTVDGGGYDVPGQSLDWTAGA
LEAIGRASREQGVDPVPVDMVGALVRRQIDEGYGSEDMSRLIESMTGDAV

SEQ SEQ ID. 5 (nucleotide sequence of imine reductase)

atgaagaccc ccgtgaccgt gctcgggctg ggtgcgatgg gacgtgcgct ggtcaccgcg ctgctcgacg cggggcggcc ggtcaccgtg tggaaccgga cgcccgggaa ggcggacgaa ctggcgggcg cggtcgaggc cgcgagcgtc cacgacgcgg tgaccgcggg cgggatcgtg gtcgtgtgcc tctacgacca cgcctcggtg cacgagacac tcgacccggt ggccgccggg ctgcgcggac gggcggtggt gaacctcacg accacgacgc ccgccgaggc acgggagctc gcggagtggg cggacggcca cggaatcgag tacctcgacg gagcgatcat ggcgacgccg ccgatgatcg gtgctccggg cgcgcagatc ctctacagcg ggtcgcgtgc ggtgttcgat gggcatcgcg aggcgctcga cctgtgggcg acgtccgtct acgacggtgc ggacgccgga atggcgtcgc tgttcgacct cgcgatcctt tcgggcatgt atccgctgtt cgccggattc ctgcacggcg ccgcgatggt ccgcgccgag ggcgtcccgg cggccgagtt cgcggagcgc gccgcgcctt tcctcgccgc gatgaccggg tcgttcgccg gcatcgcgcg aaccgtggac ggcggcggct acgacgtgcc ggggcagagc ctcgactgga cggccggcgc gctggaggcc atcggccgtg cgagccgcga gcagggcgtc gatccggttc cggtcgacat ggtggggggcg ctcgtccggc ggcagatcga cgaagggtac gggagcgaag atatgtcccg gctcatcgag agcatgaccg gtgacgcggt gtga

SEQ ID NO. 6 (nucleotide sequence of imine reductase mutant G43V)

atgaagaccc ccgtgaccgt gctcgggctg ggtgcgatgg gacgtgcgct ggtcaccgcg ctgctcgacg cggggcggtc ggtcaccgtg tggaaccgga cgcccgggaa ggcggacgaa ctggcgggcg cggtcgaggc cgcgagcgtc cacgacgcgg tgaccgcggg cgggatcgtg gtcgtgtgcc tctacgacca cgcctcggtg cacgagacac tcgacccggt ggccgccggg ctgcgcggac gggcggtggt gaacctcacg accacgacgc ccgccgaggc acgggagctc gcggagtggg cggacggcca cggaatcgag tacctcgacg gagcgatcat ggcgacgccg ccgatgatcg gtgctccggg cgcgcagatc ctctacagcg ggtcgcgtgc ggtgttcgat gggcatcgcg aggcgctcga cctgtgggcg acgtccgtct acgacggtgc ggacgccgga atggcgtcgc tgttcgacct cgcgatcctt tcgggcatgt atccgctgtt cgccggattc ctgcacggcg ccgcgatggt ccgcgccgag ggcgtcccgg cggccgagtt cgcggagcgc gccgcgcctt tcctcgccgc gatgaccggg tcgttcgccg gcatcgcgcg aaccgtggac ggcggcggct acgacgtgcc ggggcagagc ctcgactgga cggccggcgc gctggaggcc atcggccgtg cgagccgcga gcagggcgtc gatccggttc cggtcgacat ggtggggggcg ctcgtccggc ggcagatcga cgaagggtac gggagcgaag atatgtcccg gctcatcgag agcatgaccg gtgacgcggt gtga

SEQ ID NO. 7 (nucleotide sequence of imine reductase mutant L89V)

atgaagaccc ccgtgaccgt gctcgggctg ggtgcgatgg gacgtgcgct ggtcaccgcg ctgctcgacg cggggcggcc ggtcaccgtg tggaaccgga cgcccgggaa ggcggacgaa ctggcgggcg cggtcgaggc cgcgagcgtc cacgacgcgg tgaccgcggg cgggatcgtg gtcgtgtgcc tctacgtcca cgcctcggtg cacgagacac tcgacccggt ggccgccggg ctgcgcggac gggcggtggt gaacctcacg accacgacgc ccgccgaggc acgggagctc gcggagtggg cggacggcca cggaatcgag tacctcgacg gagcgatcat ggcgacgccg ccgatgatcg gtgctccggg cgcgcagatc ctctacagcg ggtcgcgtgc ggtgttcgat

gggcatcgcg aggcgctcga cctgtgggcg acgtccgtct acgacggtgc ggacgccgga atggcgtcgc tgttcgacct cgcgatcctt tcgggcatgt atccgctgtt cgccggattc ctgcacggcg ccgcgatggt ccgcgccgag ggcgtcccgg cggccgagtt cgcggagcgc gccgcgcctt tcctcgccgc gatgaccggg tcgttcgccg gcatcgcgcg aaccgtggac ggcggcggct acgacgtgcc ggggcagagc ctcgactgga cggccggcgc gctggaggcc atcggccgtg cgagccgcga gcagggcgtc gatccggttc cggtcgacat ggtgggggcg ctcgtccggc ggcagatcga cgaagggtac gggagcgaag atatgtcccg gctcatcgag agcatgaccg gtgacgcggt gtga

SEQ SEQ ID. 8 (nucleotide sequence of imine reductase mutant G43V-L89V)

atgaagaccc ccgtgaccgt gctcgggctg ggtgcgatgg acgtgcgct ggtcaccgcg ctgctcgacg cggggcggtc ggtcaccgtg tggaaccgga cgcccgggaa ggcggacgaa ctggcgggcg cggtcgaggc cgcgagcgtc cacgacgcgg tgaccgcggg cgggatcgtg gtcgtgtgcc tctacgtcca cgcctcggtg cacgagacac tcgacccggt ggccgccggg ctgcgcggac gggcggtggt gaacctcacg accacgacgc ccgccgaggc acgggagctc gcggagtggg cggacggcca cggaatcgag tacctcgacg gagcgatcat ggcgacgccg ccgatgatcg gtgctccggg cgcgcagatc ctctacagcg ggtcgcgtgc ggtgttcgat gggcatcgcg aggcgctcga cctgtgggcg acgtccgtct acgacggtgc ggacgccgga atggcgtcgc tgttcgacct cgcgatcctt tcgggcatgt atccgctgtt cgccggattc ctgcacggcg ccgcgatggt ccgcgccgag ggcgtcccgg cggccgagtt cgcggagcgc gccgcgcctt tcctcgccgc gatgaccggg tcgttcgccg gcatcgcgcg aaccgtggac ggcggcggct acgacgtgcc ggggcagagc ctcgactgga cggccggcgc gctggaggcc atcggccgtg cgagccgcga gcagggcgtc gatccggttc cggtcgacat ggtgggggcg ctcgtccggc ggcagatcga cgaagggtac gggagcgaag atatgtcccg gctcatcgag agcatgaccg gtgacgcggt gtga

SEQ ID NO. 9 (amino acid sequence of commercially available glucose dehydrogenase for reaction)

MYPDLKGKVVAITGAASGLGKAMAIRFGKEQAKVVINYYSNKQDPNEVKEEVIKAGGEAV VVQGDVTKEEDVKNIVQTAIKEFGTLDIMINNAGLENPVPSHEMPLKDWDKVIGTNLTGA FLGSREAIKYFVENDIKGNVINMSSVHEVIPWPLFVHYAASKGGIKLMTETLALEYAPKGIR VNNIGPGAINTPINAEKFADPKQKADVESMIPMGYIGEPEEIAAVAAWLASKEASYVTGITL FADGGMTQYPSFQAGRG

SEQ ID NO. 10 (nucleotide sequence of commercially available glucose dehydrogenase for reaction)

atgtatccgg atctgaaagg caaggtggtg gccattaccg gtgccgcgag tggtctgggt aaagcgatgg

ccatccgctt cggcaaggag caagccaagg tggtgatcaa ctactacagc aacaagcaag atccgaacga

agtgaaagaa gaagtgatca aagccggtgg tgaagcggtt gtggttcaag gcgacgtgac caaagaagaa

gacgtgaaga acatcgtgca gacggcgatc aaggagttcg gcacgctgga catcatgatc aacaacgccg

gtctggaaaa cccagtgcca agccacgaga tgccgctgaa ggattgggat aaggtgatcg gcaccaatct

gacgggcgcg tttctgggta gccgtgaggc gatcaagtac ttcgtggaga acgacatcaa gggcaacgtg

attaacatga gcagcgtgca cgaggtgatc ccgtggccgc tgttcgtgca ttacgccgcg agcaagggcg

gcattaaact catgaccgaa acgctggcgc tggaatatgc cccgaaaggc atccgcgtga ataacatcgg

cccgggcgcc atcaacaccc cgatcaatgc ggagaagttc gccgatccga aacagaaggc cgacgtggaa

agcatgatcc caatgggcta catcggcgaa ccagaggaaa ttgcggccgt tgccgcgtgg ctggccagca

aagaagcgag ctacgttacc ggtatcacgc tgtttgccga tggcggcatg acgcagtatc cgagtttcca

agccggtcgc ggctaa

Despite the description of the specific implementations of the present invention for the illustrative purposes, those skilled in the art should understand that various amendments can be made to these implementations without departing from the spirit and scope of the present invention. Therefore, the specific implementations and embodiments of the present invention shall not be considered as limiting the scope of the present invention. The present disclosure is limited only by the appended claims. All the references cited in the present application are incorporated herein by reference in their entireties.

## Claims

1. An imine reductase mutant, which has an amino acid sequence derived by a mutation occurring to an amino acid sequence as set forth in SEQ ID NO. 1, and an optional mutation site includes one of the following sites: position 44 with G mutating into V, or position 89 with L mutating into V, or positions 44 and 89 with G and L simultaneously mutating into V, respectively.

2. The imine reductase mutant according to claim 1, wherein the amino acid sequence is as set forth in SEQ ID NO. 2.

3. The imine reductase mutant according to claim 1, wherein the amino acid sequence is as set forth in SEQ ID NO. 3.

4. The imine reductase mutant according to claim 1, wherein the amino acid sequence is as set forth in SEQ ID NO. 4.

5. A recombinant plasmid comprising a coding gene of the imine reductase mutant of claim 1.

6. A host cell comprising the recombinant plasmid of claim 5.

7. Application of the imine reductase mutant of claim 1 in synthesis of an intermediate (S)-2-methyl-5-(pyrrol-2-yl) pyridine.

8. An application method for an imine reductase mutant in synthesis of an intermediate (S)-2-methyl-5-(pyrrol-2-yl) pyridine, comprising: binding the imine reductase mutant of claim 1 to glucose dehydrogenase to prepare the intermediate (S)-2-methyl-5-(pyrrol-2-yl)pyridine through asymmetric hydrogenation reduction by taking β-nicotinamide adenine dinucleotide disodium salt as a coenzyme and 5-(3,4-dihydro-2H-pyrrol-5-yl)-2-methylpyridine as a substrate.

9. The application method according to claim 8, wherein during the preparation of the intermediate (S)-2-methyl-5-(pyrrol-2-yl)pyridine through asymmetric hydrogenation reduction, a pH value is 6.8-8.0.

10. The application method according to claim 8, wherein during the preparation of the intermediate (S)-2-methyl-5-(pyrrol-2-yl)pyridine through asymmetric hydrogenation reduction, a reaction temperature is 15-35°C.

## INTERNATIONAL SEARCH REPORT

| International application No. |
| --- |
| **PCT/CN2023/107618** |

### A. CLASSIFICATION OF SUBJECT MATTER

C12N9/06(2006.01)i; C12N15/70(2006.01)i; C12N1/21(2006.01)i; C12P17/10(2006.01)i; C12R1/19(2006.01)i

According to International Patent Classification (IPC) or to both national classification and IPC

### B. FIELDS SEARCHED

Minimum documentation searched (classification system followed by classification symbols)

C12N C12P

Documentation searched other than minimum documentation to the extent that such documents are included in the fields searched

Electronic data base consulted during the international search (name of data base and, where practicable, search terms used)

CNTXT, DWPI, VEN, ENTXTC, CNKI, 万方数据资源系统, Wanfang Data, PubMed, ISI web of knowledge: 浙江安诺和, 亚胺还原酶, 43, 89, G43, L89, G43V, L89V, imine reductase, mutant; GenBank, 中国专利生物序列检索, China Patents Biological Sequence Search, EBI-EMBL, STN和序列: SEQ ID NO: 1-4, STN and sequences: SEQ ID NO: 1-4.

### C. DOCUMENTS CONSIDERED TO BE RELEVANT

| Category* | Citation of document, with indication, where appropriate, of the relevant passages | Relevant to claim No. |
| --- | --- | --- |
| PX | CN 115948360 A (ZHEJIANG ANNUOHE BIOLOGICAL MEDICINE CO., LTD.) 11 April 2023 (2023-04-11) description, paragraphs [0004]-[0020] | 1-10 |
| A | CN 113774036 A (DIJIA PHARMACEUTICAL GROUP CO., LTD.) 10 December 2021 (2021-12-10) description, paragraphs [0004]-[0019] | 1-10 |
| A | CN 109355266 A (EAST CHINA UNIVERSITY OF SCIENCE AND TECHNOLOGY et al.) 19 February 2019 (2019-02-19) description, paragraphs [0006]-[0054] | 1-10 |
| A | CN 114774383 A (SHANGHAI UNIVERSITY OF MEDICAL & HEALTH SCIENCES) 22 July 2022 (2022-07-22) description, paragraphs [0006]-[0019] | 1-10 |
| A | CN 102203244 A (DAICEL CHEMICAL INDUSTRIES, LTD.) 28 September 2011 (2011-09-28) description, paragraphs [0027]-[0141] | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **25 October 2023** | **10 November 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

EP 4 592 385 A1

**INTERNATIONAL SEARCH REPORT**

International application No.

**PCT/CN2023/107618**

| C. | DOCUMENTS CONSIDERED TO BE RELEVANT | |
|---|---|---|
| Category* | Citation of document, with indication, where appropriate, of the relevant passages | Relevant to claim No. |
| A | WO 2022268006 A1 (CHEN ZECONG) 29 December 2022 (2022-12-29)<br>description, paragraphs [0010]-[0065] | 1-10 |

Form PCT/ISA/210 (second sheet) (July 2022)

## INTERNATIONAL SEARCH REPORT

| International application No. |
| --- |
| **PCT/CN2023/107618** |

| **Box No. I** | **Nucleotide and/or amino acid sequence(s) (Continuation of item 1.c of the first sheet)** |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2023/107618**

| Patent document cited in search report | | | Publication date (day/month/year) | Patent family member(s) | | | Publication date (day/month/year) |
|---|---|---|---|---|---|---|---|
| CN | 115948360 | A | 11 April 2023 | None | | | |
| CN | 113774036 | A | 10 December 2021 | None | | | |
| CN | 109355266 | A | 19 February 2019 | None | | | |
| CN | 114774383 | A | 22 July 2022 | None | | | |
| CN | 102203244 | A | 28 September 2011 | US | 2011287494 | A1 | 24 November 2011 |
| | | | | JPWO | 2010024444 | A1 | 26 January 2012 |
| | | | | KR | 20110049906 | A | 12 May 2011 |
| | | | | WO | 2010024444 | A1 | 04 March 2010 |
| | | | | EP | 2330190 | A1 | 08 June 2011 |
| WO | 2022268006 | A1 | 29 December 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Neuropharmacology*, 1995, vol. 34, 563
- *Chem Eng. News*, 2000, vol. 78, 23-26
- *J. Med. Chem.*, 1997, vol. 40, 4169
- *J.Pharm.Exp.Ther.*, 2000, vol. 292, 461
- *j. org. chem.*, 1999, vol. 64, 5299
- **DUKAT et al.** *Eur. J. Med. Chem.*, 1999, vol. 34, 31